# EUROPEAN PATENT APPLICATION

(11) **EP 3 385 701 A1**
(43) Date of publication of application: **10.10.2018**
(21) Application number: 17747326.1
(22) Date of filing: 27.01.2017
(51) Int. Cl.: G01N 21/17, E03D 9/00

(54) **TOILET DEVICE**

(30) Priority: 04.02.2016 JP 2016019763
(71) Applicant: Lixil Corporation, Koto-ku Tokyo 136-8535 (JP)
(72) Inventor: OGURI, Koji, Handa-shi Aichi 475-0873 (JP); UEDA, Emi, Tokyo 136-8535 (JP); MAKI, Michitarou, Tokyo 136-8535 (JP); MIZUNO, Haruyuki, Tokyo 136-8535 (JP); KAWANAKA, Haruki, Nagakute-shi Aichi 480-1198 (JP); ISOMURA, Atsushi, Nagakute-shi Aichi 480-1198 (JP); HONDA, Chizuru, Nagakute-shi Aichi 480-1198 (JP)
(74) Representative: Müller-Boré & Partner Patentanwälte PartG mbB
(86) International application number: PCT/JP2017/003010
(87) International publication number: WO 2017/135169

(57) **Abstract**

Provided is a toilet device that obtains a more detailed estimation result of a health condition. A toilet device (1) includes a toilet body (10) that includes a toilet bowl part (11), a camera (20) that photographs feces discharged and falling into the toilet bowl part (11) in time series and acquires a plurality of still images of the feces, and a fecal properties estimation part (30) that estimates a change in property of the feces from the plurality of still images acquired by the camera (20). The toilet device (1) estimates the change in the property of the feces from the still images of the feces photographed in time series by the camera (20). Therefore, it is possible to obtain more detailed information on a user's health condition, in particular, the health condition of a digestive system (an intestinal environment).

## Description

### TECHNICAL FIELD

The present invention relates to a toilet device.

### BACKGROUND ART

Excrement of a living body such as a human body is reflected by the health condition of the living body. In particular, feces are reflected by the health condition of a digestive system (an intestinal environment). Therefore, grasping the property of feces is useful for grasping the intestinal environment. For this reason, feces have been conventionally photographed using a camera to estimate the property of the feces (see, for example, Patent Literature 1).

A device of Patent Literature 1 acquires biological data to estimate the health condition. This device includes an image photographing part and a data analyzing part. The image photographing part is provided in a toilet bowl in a toilet, and photographs the excrement of a living body. The data analyzing part analyzes the images photographed by the image photographing part. The data analyzing part analyzes the photographed images to acquire biological data related to the property of the excrement, and estimates the health condition of the living body from the acquired biological data.

### CITATIONS LIST

### PATENT LITERATURE

Patent Literature 1: Japanese Unexamined Patent Application Publication No. 2007-252805

### SUMMARY OF INVENTION

### TECHNICAL PROBLEMS

However, the device of Patent Literature 1 cannot be said to provide sufficient data. For this reason, a technique capable of estimating it in more detail has been desired.

The present invention has been made in view of the above conventional circumstances, and aims to provide a toilet device that can obtain a more detailed estimation result of a health condition (an intestinal environment).

### SOLUTIONS TO PROBLEMS

A toilet device according to the present invention includes: a toilet body that includes a toilet bowl part; a camera that photographs feces discharged and falling into the toilet bowl part in time series and acquires a plurality of still images of the feces; and a fecal properties estimation part that estimates a change in property of the feces from the plurality of still images acquired by the camera.

In this toilet device, the plurality of still images of the feces are photographed in time series by the camera. From the plurality of still images, the change in the property of the feces is estimated. That is, the toilet device estimates the time-series change in the property of the feces during single bowel motion. Therefore, it is possible to obtain more detailed information on a user's health condition, in particular, the health condition of a digestive system (the intestinal environment).

The term "falling" as described above means a time after the feces are discharged to the outside of a body until the feces reach the water surface of a reservoir part provided in a lower part of the toilet bowl part or a wall surface of the toilet bowl part.

### BRIEF DESCRIPTION OF DRAWINGS

Fig. 1 is a cross-sectional view schematically showing a toilet device according to an embodiment.
Fig. 2 shows examples of still images photographed in time series.
Fig. 3 shows examples of pattern classifications of the property of feces.

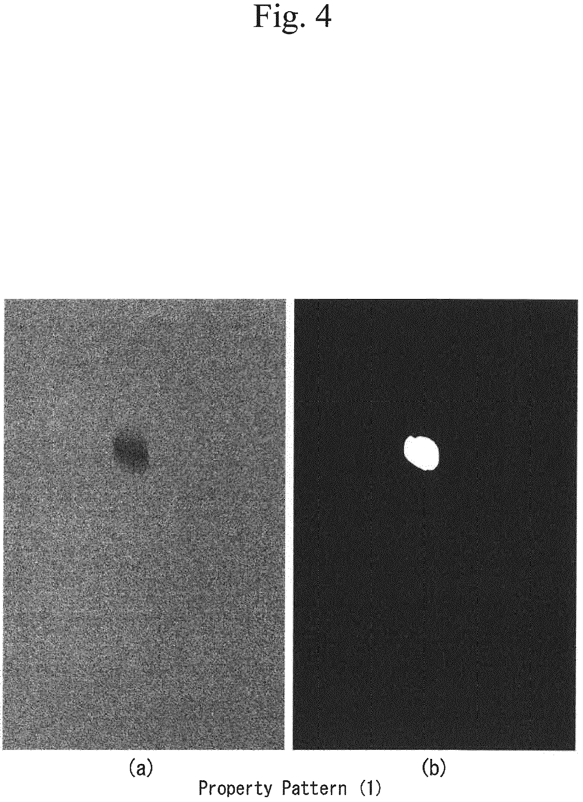

Fig. 4 shows examples of images of feces having a property pattern (1) according to the embodiment; (a) shows an original image; and (b) shows an image after a binarization processing.

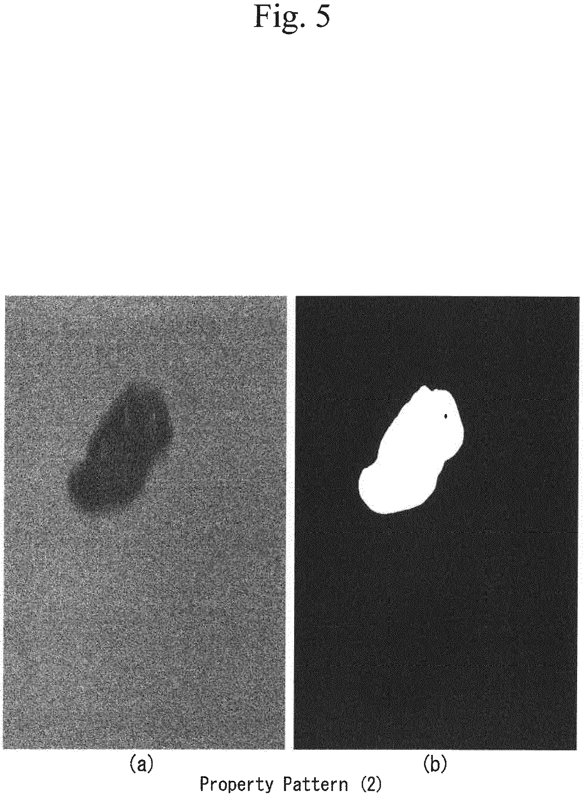

Fig. 5 shows examples of images of feces having a property pattern (2) according to the embodiment; (a) shows an original image and (b) shows an image after a binarization processing.

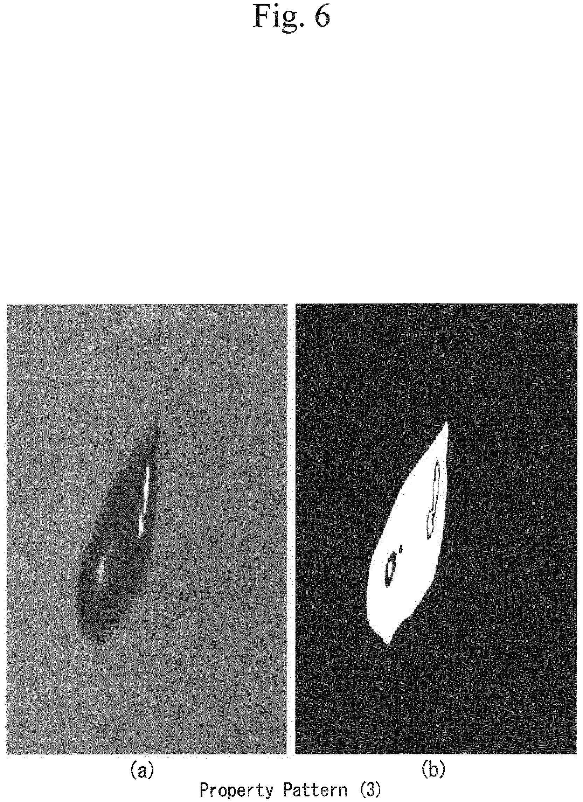

Fig. 6 shows examples of images of feces having a property pattern (3) according to the embodiment; (a) shows an original image and (b) shows an image after a binarization processing.

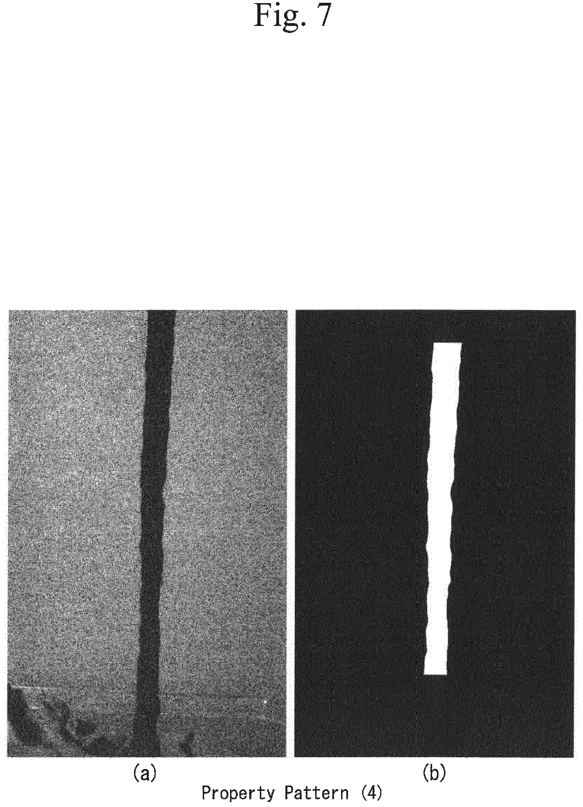

Fig. 7 shows examples of images of feces having a property pattern (4) according to the embodiment; (a) shows an original image and (b) shows an image after a binarization processing

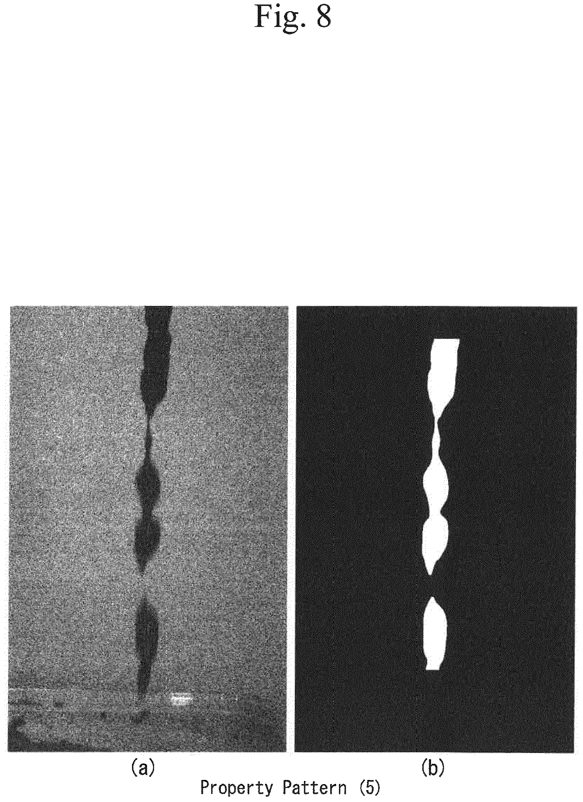

Fig. 8 shows examples of images of feces having a property pattern (5) according to the embodiment; (a) shows an original image and (b) shows an image after a binarization processing

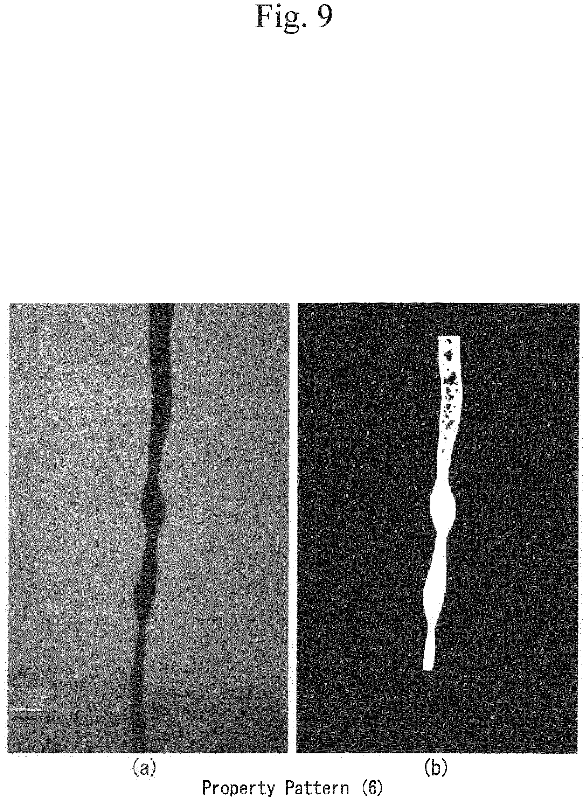

Fig. 9 shows examples of images of feces having a property pattern (6) according to the embodiment; (a) shows an original image and (b) shows an image after a binarization processing
Fig. 10 is a view for explaining estimation of a time-series change in the property of feces according to the embodiment.
Fig. 11 is a view for explaining a toilet device according to another embodiment.
Fig. 12 is a view for explaining a toilet device according to another embodiment.
Fig. 13 is a view for explaining a toilet device according to another embodiment.
Fig. 14 is a view for explaining a toilet device according to another embodiment.
Fig. 15 is a view for explaining a toilet device according to another embodiment.
Fig. 16 is a view for explaining a toilet device according to another embodiment.
Fig. 17 is a view for explaining a toilet device according to another embodiment.

### DESCRIPTION OF EMBODIMENTS

Preferred embodiments of the present invention will be described.

In a toilet device of the present invention, the fecal properties estimation part may estimate the property of the feces for each of the still images. In this case, it is possible to easily estimate a change in the property of the feces coming out while changing its shape during single bowel motion.

In the toilet device of the present invention, the fecal properties estimation part may estimate the property of the feces as one of a plurality of pre-classified property patterns. In this case, the time-series change in the property of the feces becomes clearer. Therefore, the change in the property of the feces can be more easily estimated.

In the toilet device of the present invention, the camera may be attached to a toilet seat device disposed above the toilet bowl part. In this case, the camera can be installed more easily than when the camera is attached to a toilet body.

In the toilet device of the present invention, the camera may be detachably attached to an upper part of the toilet bowl part. In this case, the camera can be attached and detached easily.

In the toilet device of the present invention, when a user's body is included in the still images, the fecal properties estimation part may perform a masking processing on a portion including an image region corresponding to the body in the still images. In this case, it is possible to protect user's privacy.

Next, an embodiment embodying the toilet device of the present invention will be described with reference to the drawings.

### <Embodiments>

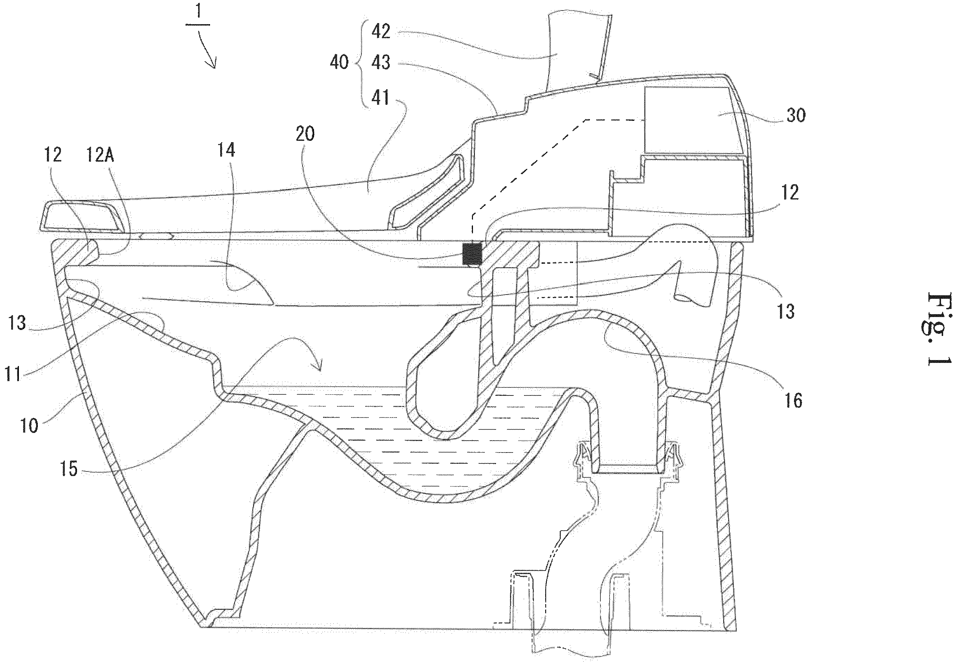

As shown in Fig. 1, a toilet device 1 of an embodiment includes a toilet body 10, a camera 20, and a fecal properties estimation part 30. The toilet body 10 includes a toilet bowl part 11. The toilet body 10 includes a rim 12 formed on an upper end of the toilet bowl part 11, and a rim water passage 13 formed below the rim 12. An opening defined by an inner peripheral surface 12A of the rim 12 is substantially elliptical in top view as seen from above the toilet body 10. The rim water passage 13 is formed in a groove shape so as to be recessed outward from the inner peripheral surface 12A of the rim 12. The rim water passage 13 is formed over substantially the entire inner periphery of the toilet bowl part 11. The toilet bowl part 11 has a spouting port 14 opened so as to be continuous with the rim water passage 13. Flush water discharged from the spouting port 14 runs in the rim water passage 13. The flush water discharged from the spouting port 14 flows along the rim water passage 13 in one circumferential direction of the toilet bowl part 11. In a lower part of the toilet bowl part 11, a reservoir part 15 in which the flush water stays is formed. A lower part of the reservoir part 15A communicates with a drainage channel 16 through which the flush water is discharged.

A toilet seat device 40 is provided above the toilet body 10. The toilet seat device 40 includes a toilet seat 41, a toilet cover 42, and a toilet seat device body 43. The toilet seat 41 has a substantially elliptical shape in top view, and is formed into an annular shape with its inside opened so that the toilet seat 41 is disposed along the upper edge part of the rim 12 of the toilet body 10. The toilet cover 42 covers the toilet seat 41 from above, and is provided to be capable of closing the openings of the toilet seat 41 and toilet bowl part 11. The toilet seat device body 43 is detachably fixed to the toilet body 10. The toilet seat device body 43 rotatably supports the toilet seat 41 and the toilet cover 42, and accommodates therein functional components (not shown) such as a private part washing device.

The camera 20 photographs feces discharged and falling into the toilet bowl part 11 in time series and acquires a plurality of still images of the feces. That is, the camera 20 photographs still images in time series of the feces after the feces are discharged until the feces fall into the reservoir part 15. The camera 20 is disposed so as to face inward toward the toilet bowl part 11 so that the feces discharged into the toilet bowl part 11 can be photographed. In the present embodiment, the camera 20 is embedded in the inner peripheral surface 12A on the backside of the rim 12 as shown in Fig. 1. That is, the camera 20 is disposed on the inner periphery on the backside of the toilet bowl part 11. The camera 20 photographs a fixed point in the toilet bowl part 11 at a predetermined angle of view at 1/60-second intervals (60 images per second).

When a body such as a user's private part is included in the still images acquired by the camera 20 in the present toilet device 1, the following processing is performed in consideration of user's privacy. That is, in the present toilet device 1, a portion including a region corresponding to the user's body in the image is subjected to a masking processing. In the subsequent processing, the image data subjected to the masking processing is used, and original image data is deleted without being left. The detection method and masking processing method of the image region corresponding to the body are not particularly limited, and can be performed by known methods.

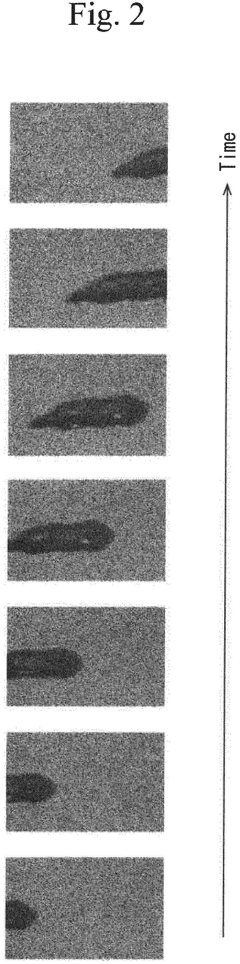

The camera 20 of the present embodiment acquires gray scale original images by monochrome photographing. Fig. 2 shows examples of a plurality of still images (original images) photographed in time series. The gray scale original images are suitable for grasping the shape of feces used for extracting feature amounts which will be described later. From the gray scale images, it is also possible to detect a color depth (shading, brightness and darkness). A camera capable of color photographing may be adopted, and in this case, its color can also be detected in addition to its shape and color depth.

A fecal properties estimation part 30 estimates a change in the property of the feces from the plurality of still images acquired by the camera 20. Specifically, the fecal properties estimation part 30 estimates the change in the property of the feces from the start of the discharge of the feces to the end thereof, based on a plurality of still images photographed in time series. In the present embodiment, the fecal properties estimation part 30 is disposed in the toilet seat device body 43 of the toilet seat device 40, but a position where the fecal properties estimation part 30 is disposed is not particularly limited.

In the present embodiment, the estimation result provided by the fecal properties estimation part 30 is data-transmitted to a display terminal (not shown) by wire or wirelessly. The user of the present toilet device 1 can confirm the estimation result using the display terminal. In the present embodiment, the display terminal can also display advice for improving fecal properties based on the estimation result provided by the fecal properties estimation part 30. The display terminal may be a terminal dedicated to and provided in the present device, or may be a smartphone, a tablet, a PC, or the like. The estimation result data may be transferred directly to the display terminal, or may be transferred via another data storage such as a cloud server. Furthermore, past estimation result data of a change in property of feces may be stored so that the estimation result data can be confirmed as needed.

The toilet device 1 having such a configuration estimates the change in the property of the feces as follows.

To estimate the change in the property of the feces, firstly, the property of the feces in each still image acquired in time series by the camera 20 is estimated. The property of the feces in the present embodiment is its hardness (softness). In the present embodiment, the hardness as a property of feces is classified into any of property patterns roughly divided into six. That is, in the present embodiment, the fecal properties estimation part 30 estimates the property of the feces as one of a plurality of pre-classified property patterns. The property of the feces is estimated for each still image.

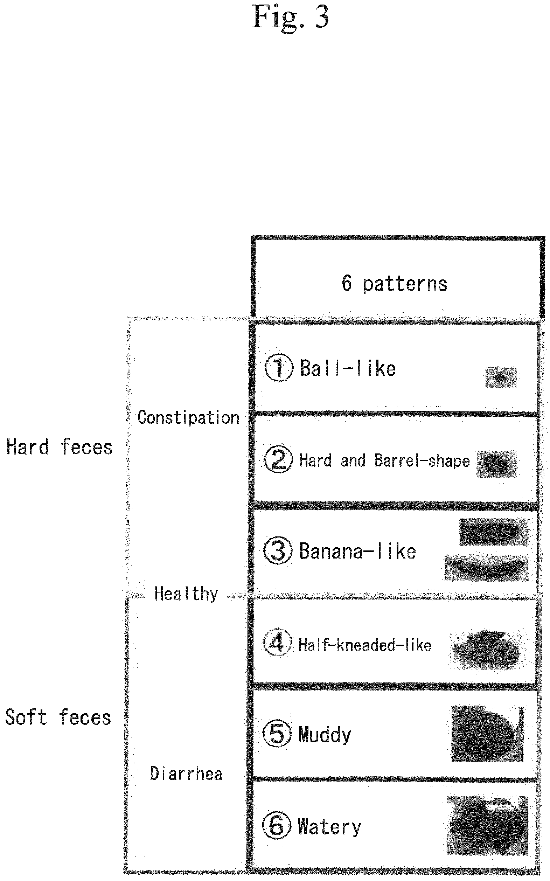

The six property patterns are (1) ball-like, (2) hard and barrel-shape, (3) banana-like, (4) half-kneaded-like, (5) muddy, and (6) watery. Among these properties, (1) and (2) are defined as properties of feces discharged in constipation condition; (3) and (4) are defined as properties of feces discharged in healthy condition; and (5) and (6) are defined as properties of feces discharged in diarrhea condition. Among the above properties, (1) to (3) are also defined as hard feces, and (4) to (6) are also defined as soft feces. Fig. 3 shows examples of the property patterns classified into six.

To estimate the hardness (softness) of the feces, it focuses on the shape of the feces. Specifically, features related to the shape are extracted from the photographed still images. That is, a plurality of feature amounts related to the shape of the feces in the still images photographed by the camera 20 are extracted, and then the property of the feces is estimated based on the plurality of feature amounts. The feature amounts to be extracted are, for example, the lateral and vertical widths of the image region corresponding to the feces in the still images, and the number of pixels constituting the image region, or the like.

The property of the feces can be estimated from the extracted feature amounts as follows, for example. Firstly, feature amounts are extracted for a plurality of feces having previously known property patterns. This makes it possible to grasp the tendency of the feature amounts in each property pattern. By comparing this tendency with the tendency indicated by the feature amounts extracted from the estimation target image of the property of the feces, which one of the property patterns of the above (1) to (6) the feces belong to is estimated.

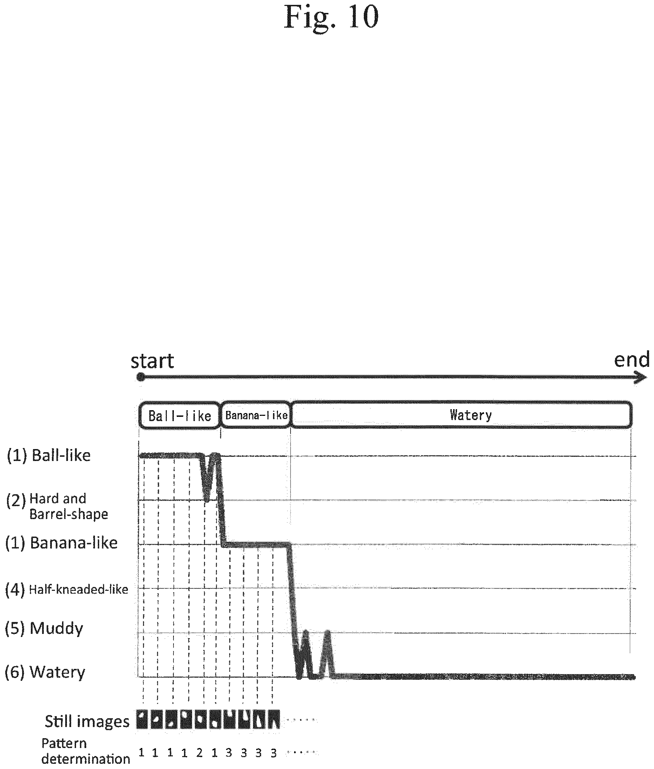

The fecal properties estimation part 30 estimates the property of the feces in each still image photographed in time series, and then estimates the change in the property of the feces. Fig. 10 shows estimation examples of the time-series change of the feces during single discharging. From Fig. 10, during single bowel motion, it is estimated that the property of the feces has changed in three stages in order of (1) ball-like, (3) banana-like, and (6) watery. It is also estimated that a period when the feces have been discharged in the property pattern of (6) water-like makes up most of the total time as compared with periods when the feces have been discharged in the property patterns of (1) ball-like and (3) banana-like. In this way, the fecal properties estimation part 30 can estimate the time-series change in the property of the feces during single bowel motion.

Accordingly, the toilet device of the embodiment can obtain a more detailed estimation result of the user's health condition.

As described above, in the toilet device 1 of the present embodiment, the estimation result of the health condition is displayed on a display terminal (not shown). The toilet device 1 of the present embodiment also gives advice for improving the property of the feces in addition to the estimation result of the health condition. The advice for improving fecal properties is displayed on the display terminal as with the estimation result.

The advice for improving fecal properties is displayed based on the estimation result of the change in the fecal properties. Examples of the advice for improving fecal properties includes, when it is estimated that the feces changing its property from (1) ball-like to (2) hard and barrel shape have been discharged during single bowel motion, "You would eat too much meat. Please ingest vegetables to take a well-balanced diet.", "If you hold it in, you miss the chance to go to the toilet, leading to constipation. Please go to the toilet as quickly as possible when you feel the need to go to the toilet", and the like. In this way, in the toilet device 1, advice related to the entire lifestyle including meals is displayed on the display terminal. The advice information may be stored together with the estimation result data so that the advice information can be confirmed as needed.

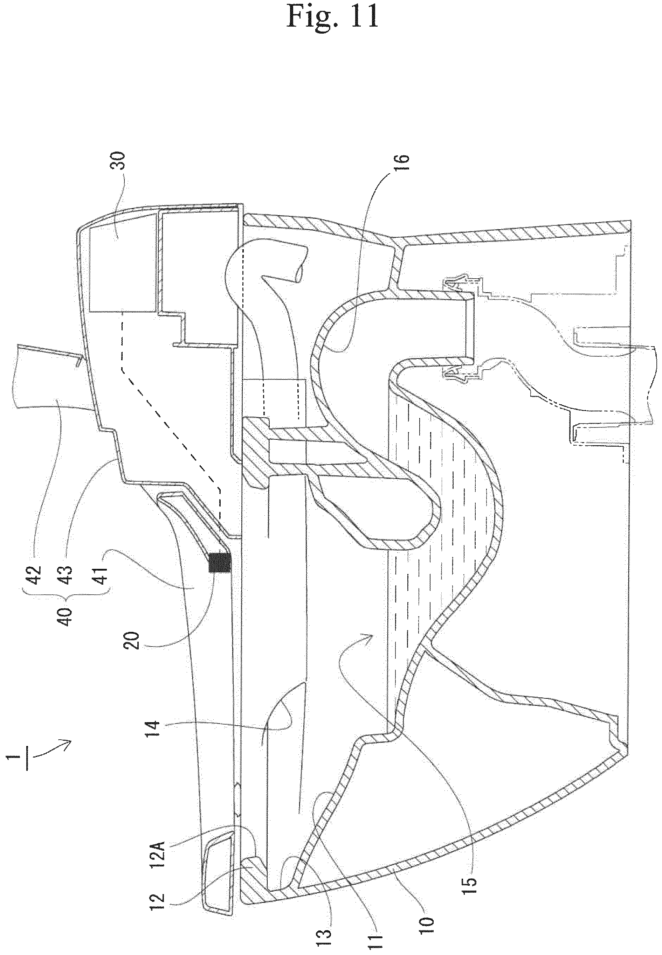

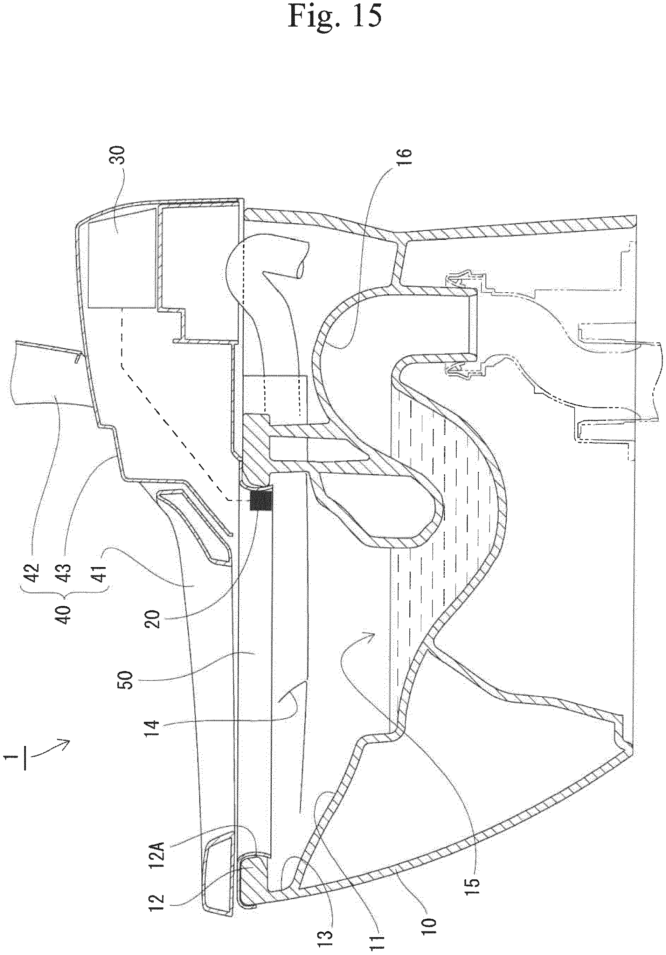

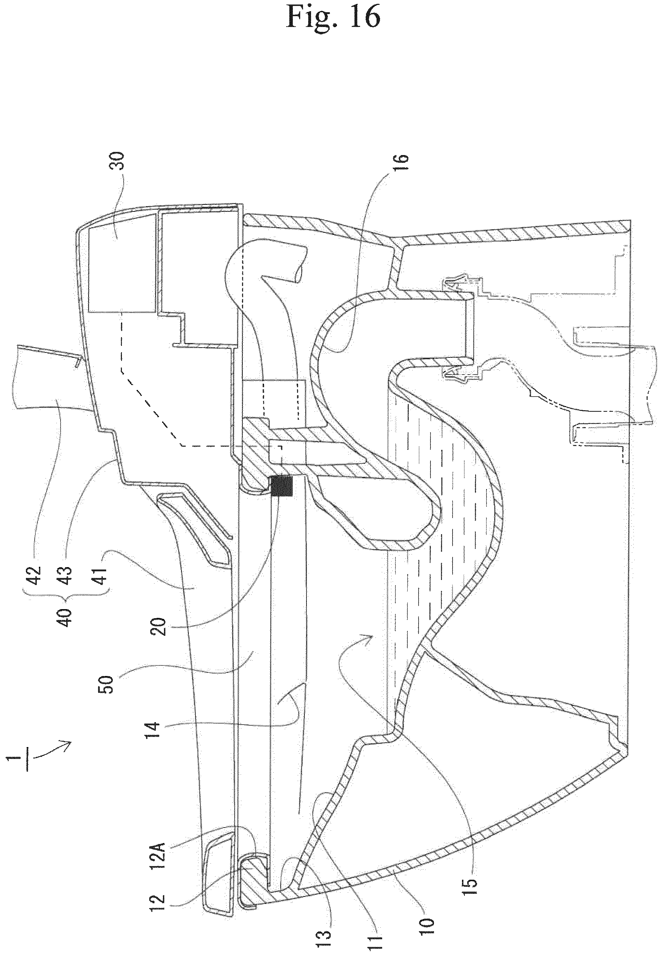

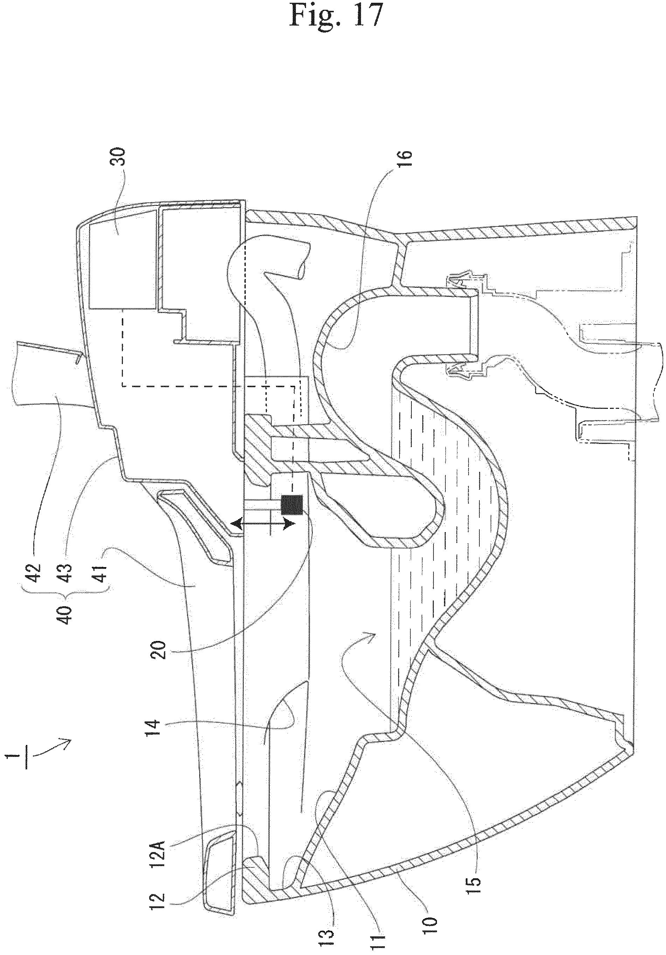

The present invention is not limited to the embodiment described in the above descriptions and drawings, and the following embodiments are also included in the technical scope of the present invention.
(1) In the embodiment, the camera is disposed so as to be embedded in the inner periphery on the backside of the toilet bowl part. However, the present invention is not limited thereto. For example, the camera may be disposed at a position other than the backside such as a front side or a lateral side of the inner periphery of the toilet bowl part. The camera may be disposed on a surface without being embedded.
(2) In the embodiment, the fecal properties estimation part estimates the property of the feces as one of the property patterns pre-classified into six. However, the present invention is not limited thereto. For example, the property of the feces may be estimated as one of the property patterns classified into 5 or less, or 7 or more.
(3) The embodiment shows an example in which the camera is disposed in the toilet bowl part. However, the present invention is not limited thereto. For example, the camera may be disposed in the toilet seat. Examples of such a configuration are shown in Figs. 11 to 14.
   Fig. 11 shows an example in which a camera is disposed on the inner periphery of a general toilet seat 41.

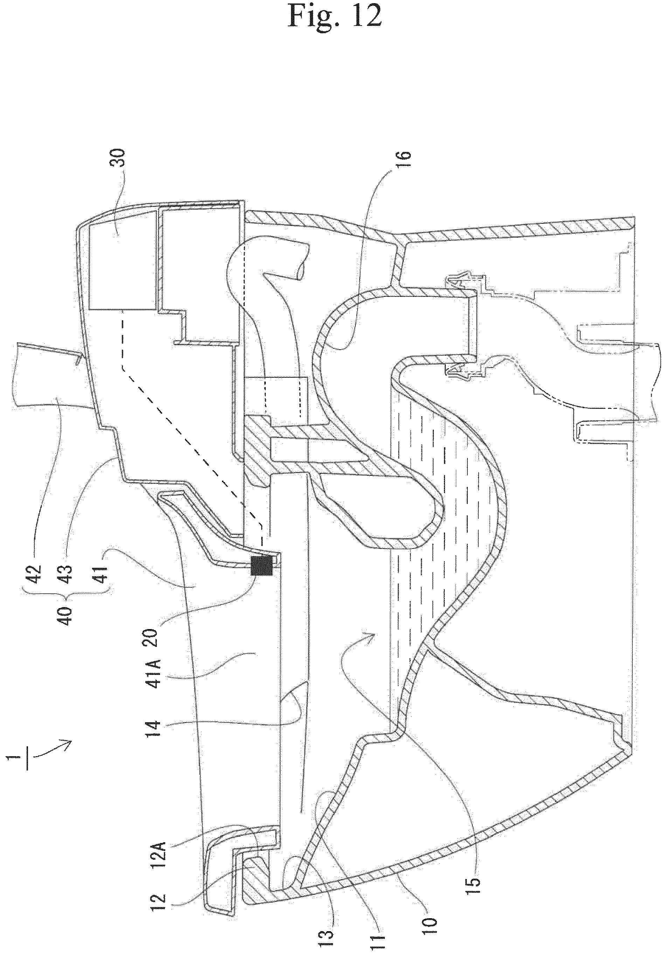

   Fig. 12 shows an example in which the toilet seat 41 includes an extended part 41A formed so that an inner peripheral side portion thereof extends downward into the toilet bowl part 11, and a camera 20 is disposed in the extended part 41A. In this case, the camera can be suitably disposed closer to the discharged feces than when the camera is disposed as shown in Fig. 11 or the like.

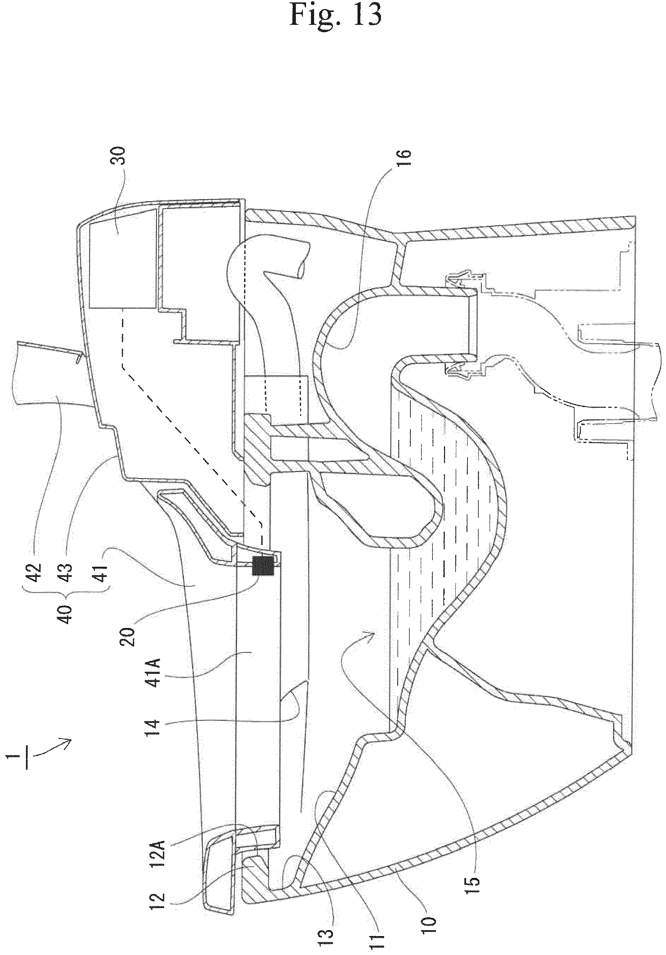

   Fig. 13 shows an example in which the toilet seat 41 includes the same extended part 41A as that in Fig. 12, but the extended part 41A is detachably provided on the toilet seat 41. In this case, the extended part 41A can also be easily attached to an existing toilet seat. The camera can be removed together with the extended part, which provides excellent maintainability.

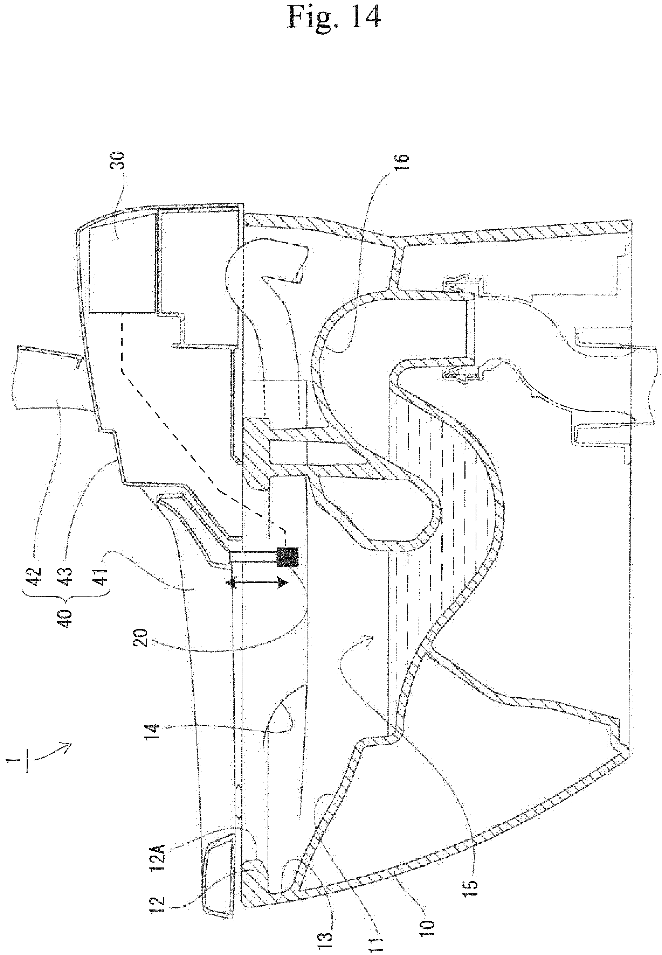

   Fig. 14 shows an example in which the camera 20 is provided extendably (protrudably) from a lower surface of the toilet seat 41 into the toilet bowl part 11. In this case, it is possible to extend the camera when the device is used, and to store the camera in the toilet seat when the device is not used. The camera may be directly attached to the lower surface of the toilet seat 41.
(4) In the embodiment, the camera is disposed on the inner periphery of the rim of the toilet bowl part. However, the present invention is not limited thereto. For example, the camera may be detachably attached to the rim. Figs. 15 and 16 show examples of a configuration in which the camera is detachably attached to the rim.
   Fig. 15 shows an example of a configuration in which the toilet device 1 includes a fixing member 50 for attaching the camera 20 in the toilet bowl part 11. The fixing member 50 is attached to an inside of the toilet bowl part 11 in such a configuration as to be suspended downward from the upper end of the rim 12. In this case, the camera can be removed together with the fixing member, which provides excellent maintainability. The camera can be attached also to an existing toilet.
   Fig. 16 shows a configuration including the same fixing member 50 as that in Fig. 15, but the camera 20 is disposed in the fixing member 50 so as to be located in the rim water passage 13. In this case, the camera can be washed with flush water flowing through the rim water passage. As a result, contaminants such as excrement adhering to the camera can be suitably washed off.
(5) In the embodiment, the camera is disposed in the toilet bowl part. However, the present invention is not limited thereto. For example, the camera may be attached to the toilet seat device body. Fig. 17 shows an example in which the camera 20 is attached to the toilet seat device body 43. In Fig. 17, the camera 20 is provided extendably (protrudably) from the lower surface of the toilet seat device body 43 into the toilet bowl part 11. In this case, it is possible to extend the camera when the device is used, and to store the camera in the toilet seat device body 43 when the device is not used. The camera may be attached directly to the lower surface of the toilet seat device body, or attached to a member extending downward from the lower surface of the toilet seat device body.

### REFERENCE SIGNS LIST

- 1:: toilet device
- 10:: toilet body
- 11:: toilet bowl part
- 12:: rim
- 12A:: inner peripheral surface of rim
- 13:: rim water passage
- 14:: spouting port
- 15:: reservoir part
- 16:: drainage channel
- 20:: camera
- 30:: fecal properties estimation part
- 40:: toilet seat device
- 41:: toilet seat
- 41A:: extended part
- 42:: toilet cover
- 43:: toilet seat device body
- 50:: fixing member

## Claims

1. A toilet device comprising:
a toilet body that includes a toilet bowl part;
a camera that photographs feces discharged and falling into the toilet bowl part in time series and acquires a plurality of still images of the feces; and
a fecal properties estimation part that estimates a change in property of the feces from the plurality of still images acquired by the camera.

2. The toilet device according to claim 1, wherein the fecal properties estimation part estimates the property of the feces for each of the still images.

3. The toilet device according to claim 2, wherein the fecal properties estimation part estimates the property of the feces as one of a plurality of pre-classified property patterns.

4. The toilet device according to any one of claims 1 to 3, wherein the camera is attached to a toilet seat device disposed above the toilet bowl part.

5. The toilet device according to any one of claims 1 to 3, wherein the camera is detachably attached to an upper part of the toilet bowl part.

6. The toilet device according to any one of claims 1 to 5, wherein the fecal properties estimation part performs a masking processing on a portion including an image region corresponding to a user's body in the still images when the user's body is included in the still images.
